# EUROPEAN PATENT SPECIFICATION

(11) **EP 0 682 696 B1**
(45) Date of publication and mention of the grant of the patent: **10.12.1997**
(21) Application number: 94905803.6
(22) Date of filing: 07.02.1994
(51) Int. Cl.: C11D 3/39, D06L 3/02, C07C 409/24, C07C 409/26, C07C 409/30, D21C 9/16, A61L 2/00

(54) **OXIDISING AGENTS**
OXYDATIONSMITTEL
AGENTS D'OXYDATION

(30) Priority: 08.02.1993 GB 9302442
(43) Date of publication of application: 22.11.1995
(73) Proprietor: Warwick International Group Limited, Holywell, Flintshire CH8 9HE (GB)
(72) Inventor: CROUD, Vincent Brian, Holywell, Clwyd CH8 7HZ (GB); TOMPSETT, Stephen James, Nr Rhyl, Clwyd LL18 1AA (GB)
(74) Representative: Jones, Helen Marjorie Meredith
(86) International application number: GB9400230
(87) International publication number: WO9418299

(56) References cited:
- EP-A- 0 092 932
- EP-A- 0 125 781
- EP-A- 0 140 648
- EP-A- 0 241 137
- EP-A- 0 253 487
- EP-A- 0 396 287
- WO-A-93/12067
- US-A- 2 955 905
- US-A- 3 551 087

## Description

The present invention relates to the in situ production of peroxygen-based oxidising species from a peroxygen source and an activator followed by the use of the product as an oxidising agent, for instance as a bleach or a biocide.

It is very well known in the laundry detergent field to use a combination of peroxygen bleach precursor (or peroxygen source) and bleach activator in the same or separate compositions. The bleach activators are acyl donors. The bleach precursor and activator, when added to the aqueous laundry liquor react together in a reaction involving attack by peroxide anion on the activator to form a peroxygen bleaching species usually the peroxy acid anion. The conditions of laundry liquors are invariably alkaline, usually having a pH of at least 9. The activator and peroxygen source do not react together during storage and are themselves stable under storage conditions.

It is known to coat or agglomerate bleach activators to increase their stability on storage in a laundry detergent composition and/or to affect their dissolution characteristics in the wash liquor. Fatty acids have been used and in WO-A-9213798 solid organic acids such as monomeric aliphatic hydroxy carboxylic acids including citric, lactic and glycolic acids, are incorporated into activator particles. In EP-A-0028432 bleach particles are stabilised for storage by incorporating acidic components. The particles are incorporated into conventional alkaline laundry detergents.

In WO-A-9312067 (unpublished at the priority date of the present case) acylated citrate esters are used to increase the bleaching effect of hydrogen peroxide. The esters are incorporated into bleach booster compositions which then appear to be used in conjunction with normal laundry detergents. The bleach booster composition may be acidic or alkaline.

In EP-A-0241137 liquid laundry bleaching compositions are described which contain a dispersion of a solid particulate bleach activator in acidic aqueous hydrogen peroxide. The preferred activators are substituted phenyl esters of alkanoic acids. The compositions are used in conjunction with conventional laundry detergents so that the detersive solution produced is alkaline.

Organic peroxy acids are well known as useful oxidising agents for a wide range of specific oxidation reactions that they perform in high-to-quantitative yield. A review of the various methods known for the preparation of peroxy acids is available in "Organic Peroxides", volume 1, D. Swern Ed, Wiley Interscience (1970) 313-335. Most of the reactions described use the corresponding carboxylic acid, the acid anhydride, the acid chloride or the aldehyde as the starting materials for instance for a perhydrolysis reaction using hydrogen peroxide. One of the reactions uses the alkaline perhydrolysis of imidazolides of carboxylic acids to form the peroxy carboxylic acids (Folli, U et al (1968) Bollettino, 26, 61-69).

In GB-A-931,119 a process for producing carboxylic peroxy acids by reacting hydrogen peroxide with an ester of the organic carboxylic acid in the absence of water and in the presence of a catalytic quantity of an acid catalyst is described. The process is used to make peracetic, perbenzoic, peradipic, perpropionic and peroxalic acids. The process requires hydrogen peroxide to be dissolved into the liquid ester, water to be removed and then acid catalyst to be added only after complete removal of the water. In one example the product solution was subsequently used to oxidise cyclohexene to form cyclohexene oxide.

GB-A-930,056 describes a process for the reaction of an aromatic carboxylic acid ester with hydrogen peroxide in an alkane sulphonic acid to form the aromatic peroxy acid. Hydrogen peroxide was added to the reaction mixture as an aqueous solution having a concentration of at least 70%.

GB-A-1,363,916 describes anhydrous processes for producing percarboxylic acids which are aromatic or aliphatic in nature by carrying out the reaction in the presence of organic phosphorous compounds. The citation contemplates the use of acid derivatives, including esters, although anhydrides or the acids themselves are preferred and there are no worked examples using other derivatives. There may be some water left in the reaction mixture.

It is known to produce peracetic acid, a strong oxidising agent, in situ by reaction of acetic acid and hydrogen peroxide, for instance to be used in epoxidation reactions. The advantage of using the peracid rather than hydrogen peroxide itself is that it is a stronger oxidising agent. Peracids are however unstable and can be dangerous to transport in bulk. The problem with the in situ reaction of acetic acid and hydrogen peroxide is that water must be removed to drive the reaction or else a large excess of one of the reactants is used, which requires complex separation and recycling steps. Acetic anhydride has also been used in place of acetic acid as starting material for this in situ reaction. The conditions during the in situ reaction step and subsequent oxidation reaction will be acidic. Acetic acid and acetic anhydride as starting materials for an in situ reaction require special precautions on handling and so are not suitable for use in a domestic environment. Acetic anhydride is water sensitive and so requires special storage conditions.

In FR-A-1176059 and FR-A-1187519 the bleaching properties of hydrogen peroxide solution at acidic pH is increased by the addition of anhydrides of organic acids such as acetic anhydride. The resultant solution is used at high temperatures, from 50°C up to over 100°C. The utility appears to be in the industrial bleaching of fabrics. GB-A-901687 and US-A-3227655 constitute similar disclosures and describe the incorporation of heavy metal sequesterants into the bleaching solution and the use of ammonia or ethanolamine to regulate the pH. In all of these disclosures the anhydride is incorporated in stoichiometric or higher amounts as compared to the amount of peroxide.

There have been descriptions of the in situ formation of peracid and the subsequent use of the solution of the peracid as a bleach under acidic conditions. However these tend to be for specialised or industrial processes and/or use activator compounds which are undesirable. For example in DE-A-2227602 dialkyl dicarbonate compounds are used to increase the bleaching effect of hydrogen peroxide at a range of pH's from acidic to alkaline. The mechanism of activation is not elucidated.

In US-A-3551087 and US-A-3374177 a process is described in which formaldehyde or a formic acid ester or formamide is reacted with hydrogen peroxide to form performic acid solution which is then used as a bleach. The reaction and the bleaching take place in an acidic environment. The bleaching process is part of the industrial dyeing process for wool and silk. Performic acid is, however, extremely unstable and even relatively dilute solutions can explode at ambient temperatures. It is furthermore corrosive and an irritant, as is formic acid, the by-product of the bleaching reaction. For these reasons formate activators are undesirable, especially for use in a domestic or other non-industrial context.

In EP-A-0545594 tooth whitening compositions are described which contain peroxy acetic acid as the bleaching agent. Compositions into which peroxy acetic acid itself is incorporated are acidic. It is suggested that the peroxy acetic acid can be generated in situ by the reaction in aqueous solution of tetraacetyl ethylene diamine and sodium perborate. In the specific examples of that embodiment of the invention the aqueous solution formed when the perborate and activator are dissolved is alkaline.

It would be desirable to find a system with the stability and advantages of the bleach precursor/activator combinations used in the laundry detergent industry, but where the reaction between precursor and activator and the subsequent oxidation (including bleaching) step are carried out under acidic conditions and at relatively low concentrations.

In a new process according to the invention a solid composite product comprising a solid peroxygen source and an activator compound which is an ester of a C₂ or higher carboxylic acid is added to water whereby the peroxygen source and activator compound are brought into contact with one another in aqueous reaction mixture in which the peroxygen source is present at a concentration of less than 10M whereby the peroxygen source is reacted with the activator compound in a first step in aqueous solution, in the reaction mixture under acidic conditions to form an oxidising species which is a stronger oxidising agent than the peroxygen source and, in a second step, the aqueous composition containing the said oxidising species which is the product of the first step is immediately used without removal of any by-products or addition of any other materials as a bleaching agent, disinfectant, sterilising agent or biocide in an aqueous environment at pH of less than 7.

In the invention the activator compound preferably has the formula I 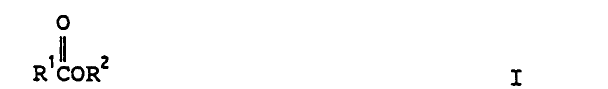 in which R¹ is an alkyl, alkenyl, aralkyl, alkaryl or aryl group, any of which groups has up to 24 carbon atoms and may be substituted or unsubstituted, and R² is selected from C₁₋₂₄-alkyl, -alkenyl, -aralkyl, -alkaryl and -aryl groups, any of which are substituted or unsubstituted, the peroxygen source being present in the reaction mixture at a concentration of less than 10M in a first step in aqueous solution under acidic conditions to form an oxidising species which is a stronger oxidising agent than the peroxygen source.

Without being bound by theory, the present inventors believe that the mechanism of reaction is that the O-acyl activator is perhydrolysed, the oxygen containing part of the molecule acting as a leaving group, to form the respective percarboxylic acid. That compound is thus believed to be a percarboxylic acid of the formula II

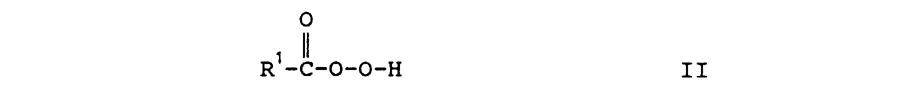

The first step is consequently referred to hereinafter as the perhydrolysis step.

It is preferred that R¹ is an aliphatic group preferably a methyl group or a C₄₋₂₄-alkyl, or -alkenyl, more preferably a C₁₋₁₈-alkyl or -alkenyl group or an aryl group.

In the present invention alkyl and alkenyl groups may be straight, branched or cyclic.

R¹ and R² may be joined to form a cyclic compound, i.e. a lactone. The cyclic group may additionally include heteroatoms, for instance oxygen or sulphur or optionally substituted nitrogen atoms. The cyclic group may be saturated or unsaturated.

Substituents can include hydroxyl,=N-R³ in which R³ is selected from any of the groups represented by R¹ and is preferably lower alkyl, lower alkenyl, amine, acyl, acyloxy, alkoxy, aryl, aroyl, aryloxy, aroyloxy, halogen, amido, and imido groups and the like as well as other groups not adversely affecting the activity of the compound and keto groups. It is important that the carbon atom adjacent to the oxygen atom in the formula I is not the carbon atom of a keto group, as compound was an anhydride and anhydrides are not covered in the new process. The activator compounds which are used in the process are generally more storage stable than anhydrides.

In the invention the activator compound can be any ester which has been described as a bleach activator for use in laundry detergents, for instance sugar esters, such as pentaacetylglucose, esters of imidic acids such as ethyl benzimidate triacylcyanurates, such as triacetylcyanurate and tribenzoylcyanurate, and esters giving relatively surface active oxidising products for instance of C₈₋₁₈-alkanoic or -aralkanoic acids such as described in GB-A-864798, GB-A-1147871 and the esters described in EP-A-98129 and EP-A-106634, for instance compounds of the formula I where R² is an aryl compound having a sulphonic acid group (optionally salified) substituted in the ring to confer water solubility on a benzyl group, especially nonanoyloxybenzenesulphonate sodium salt (NOBS), isononanoyloxybenzenesulphonate sodium salt (ISONOBS) and benzoyloxybenzenesulphonate sodium salt (BOBS) or phenyl esters of C₁₄₋₂₂-alkanoic or -alkenoic acids, esters of hydroxylamine, geminal diesters of lower alkanoic acids and gem-diols, such as those described in EP-A-0125781 especially 1,1,5-triacetoxypent-4-ene and 1,1,5,5-tetraacetoxypentane and the corresponding butene and butane compounds, ethylidene benzoate acetate and bis(ethylidene acetate) adipate and enol esters, for instance as described in EP-A-0140648 and EP-A-0092932.

In the process of the invention the precursor peroxygen source may be an inorganic persalt, for instance a percarbonate or, a perborate, for instance sodium perborate, or an organic peroxide such as benzoyl peroxide or urea peroxide.

The pH in the perhydrolysis step is preferably less than 6.5 throughout the entire reaction. Since the pH tends to drop during the reaction due to the formation of the percarboxylic acid, this means that the pH should be acidic and preferably less than 6.5 at the beginning of the reaction. Preferably the pH is less than about 6.0, at the start of the reaction. The pH is usually more than 2.0, at the start of the reaction and preferably throughout the reaction, preferably more than 5.0.

In the perhydrolysis reaction the amount of water present is preferably at least as much as (in terms of moles) as the peroxgyen source. The total concentration of peroxygen source is less than 10M in the reaction mixture. The concentration of peroxygen source is preferably such as to give the equivalent available oxygen as concentrations of hydrogen peroxide less than 30% w/v (that is weight of hydrogen peroxide based on volume of water plus hydrogen peroxide and any other reactants present). Where the product of the reaction is to be used in a domestic environment or other environment where it is difficult to take special precautions in handling the products, it is preferred for the concentration to be equivalent to a hydrogen peroxide concentration not less than 20 or less than 15 or even 10% w/v for instance 5% w/v or less. The concentration is usually at least 0.2%, preferably at least 1% w/v, more preferably at least 2% w/v. For instance, the concentration of perborate or percarbonate is suitably in the range 0.01M to 10M, preferably in the range 0.05 or 0.1M to 5M, more preferably in the range 0.2M to 2M, often less than 1M, in the perhydrolysis reaction mixture.

In the perhydrolysis step of the reaction the temperature is preferably in the range 0 to 95°C, more preferably in the range 10 to 80°C. The invention is most useful when the temperature is less than 60°C, or even less than 50°C, for instance less than 40°C or even around room temperature. The temperature is often above 20°C. The temperature in any subsequent oxidising step is preferably in the same ranges as the temperature during the perhydrolysis step and is preferably substantially the same temperature especially where the product solution is immediately used for instance as a bleach or disinfectant. A particular benefit of using activator in conjunction with peroxygen bleach is that the oxidising product is produced and is active at a relatively low temperature, for instance less than hand-hot which is advantageous in terms of user safety.

The activator compound is generally used in an amount such that it is capable of providing in the range 0.1 to 5.0 equivalents of acyl groups based on the moles of peroxygen source, preferably in the range 0.2 to 1.0. The amount is often less than stoichiometric, for instance up to 0.9 or 0.8 of the stoichiometric amount for total reaction. Often less than this is used although the optimum amount depends upon the degree of increase in oxidising performance compared to the costs of the peroxygen source and activator.

The present invention provides also a new use of a composite product comprising starting materials for the perhydrolysis reaction. Preferably the product can simply be added to water to provide the entire reaction mixture. The product therefore comprises a peroxygen source, a compound of the formula I as well as, if necessary, components for rendering the pH of an aqueous solution to which the components of the product are added acidic. In some circumstances additional acidifying components are not necessary since the peroxygen source may, itself be sufficiently acidic for the desired pH to be achieved.

An acidifying component (acid generating species) may comprise an acid and/or buffering material. The component may comprise a polybasic organic acid, such as a polybasic carboxylic acid such as citric, succinic, or adipic acid or sulphamic acid. Alternatively the component may react with a by-product of the perhydrolysis reaction to make an acid. Where perborate is used, borate is a by-product and so any component known to react with borate to drop the pH, eg cis-1,2 diols, eg glycols and polyols, boric acid or sodium dihydrogen phosphate, can be used. Such acidifying components are also suitable for use with the other inorganic persalts. The acidifying component used is preferably such as to give a pH of less than 6.5 when the product is dissolved in water to produce a predetermined concentration of peroxygen source in the range 0.1M to 5M, preferably in the range 0.2 to 2.0M.

Although the composite product may contain the individual components in each separate compositions, for instance one of which contains the peroxygen source and another of which contains the activator of the formula I, and another of which contains an acidifying component, if any, it is preferred to provide at least the activator and acidifying component in a mixture as a single composition in a form in which they are stable. Such a product which does not contain peroxygen source may, for instance, be added to an aqueous solution of peroxygen source such as hydrogen peroxide, which is readily commercially available in the form of, for instance, 60%, 20%, 10% or, preferably, 5% w/v solutions. It is preferred for all of the components to be provided in a single composition, in which the components do not react, and which is therefore substantially waterfree.

The or each composition forming the composite product is in solid form, for instance as a mixture of particles of the individual components or, more preferably, comprising particles each of which comprise all of the components. Such particles may be provided by techniques similar to those used in the laundry detergent industry, for instance including particles produced by spray drying liquid slurries, by granulation techniques using binders (for instance synthetic or natural polymers or derivatives) or by melt blending followed by extrusion or other techniques.

Preferably the product contains the active ingredients in appropriate relative quantities so that when the composition is diluted (or the compositions are mixed) with water the first step of the reaction proceeds at the optimal rate and at the desired pH. The activator and peroxygen source are for instance present in relative amounts such that 45% to 150% of the stoichiometric amount of activator (for complete reaction with the peroxygen source) is provided. Preferably the amount of activator is 10 to 100%, more preferably 20 to 80% of the stoichiometric amount.

The composite product may include other additives, for instance stabilisers which stabilise the product before use, as well as stabilisers for the peroxy acid oxidising species formed in the reaction, especially heavy metal sequestrants. The new product may also include surfactants to act as wetting agents and inorganic salts, for instance which affect the physical properties of the solid form or act as diluents. Other ingredients may be included depending on the form of the product and the final application of the reaction product, for instance perfumes.

Sometimes it may be desirable to add additional ingredients for the second step such as pH-adjusters, surfactants/wetting agents which may be cationic, anionic, amphoteric, non-ionic, or other additives to improve the second step of the process, for instance co-disinfectants, biocides, slimicides, enzymes, enzyme inhibitors or radical scavengers, abrasives etc. Cobiocides are particularly valuable where the primary objective of the second step is disinfectional sterilisation.

The reaction product of the perhydrolysis reaction is used immediately, without removal of any by-products or addition of other materials, in a second step in which it is used as an oxidising agent, bleaching or disinfecting agent.

The second step of the process of the present invention may be used as a bleaching/disinfection process, by which we mean any process in which unwanted colour is reduced or removed, non-coloured stains are reduced or removed and/or a substrate is disinfected. For instance the second step may include processes in which hard surfaces (eg floors, food preparation surfaces, utensils, toilets, washing facilities) in domestic, industrial or institutional environments are cleansed, for bleaching or other oxidative treatments used in fibre and fabric (for instance during fabric manufacture and dyeing) or the solution is used in water, effluent or sewage treatment as a biocide, in pulp and paper bleaching, paper deinking, wood bleaching, as a biocide, fungicide, bacteriocide, sporicide or viricide, as a contact lens disinfectant or general disinfectant for use inter alia in general environmental clean up. Furthermore the second step may be used in food production for instance to bleach flour, beverages, or edible oils in the food and brewing industries, for instance to clean pipes used for beverages, or, in cosmetic uses such as hair bleaching or tooth or denture whitening and disinfecting.

Since the reaction can be carried out at a relatively low concentration it can be carried out without special precautions, for instance in a domestic or institutional environment.

Compositions which are suitable to be diluted direct into water to allow the first and second steps of the reaction to proceed without further additions, may be categorised in two convenient categories.

The first category of composition is in solid form and includes a surfactant. These compositions will be suitable for use as hard surface cleaners and other uses where surface active disinfection/bleaching is required, for instance floor cleaning compositions, domestic and institutional hard surface cleaners, toilet disinfectants, general toiletries disinfectant, sanitising bottles, including glass and plastic bottles, and pipe cleaning compositions. For most of these uses it will be desirable for the composition to be relatively low foaming, although for some, for instance toilet disinfecting and general toiletries disinfectant, it may be desirable for the composition to have a relatively high foam. The use of suitable surfactants which will foam is well known in the art. For compositions which are desired to be low foam, it may desirable to incorporate anti-foaming agents, for instance soap or silicone anti-foams. Formulations including surfactants may be useful in other applications such as for use to bleach fibres or fabrics, such as nappies or in fabric production, cellulose fibres, especially in paper de-inking operations, and in general environmental clean-up operations.

The second category of formulation comprises a solid composition but without surfactant. These may be useful where no surface activity is necessary, for instance in effluent and water treatment, in toilet disinfectants, for use as a swimming pool treatment, for colour removal from chemicals, from pulp during paper making or recycling, in general industrial sterilisation and in some domestic sterilisation situations, for instance as a general toiletry disinfectant, in denture cleaning compositions, in sanitising glass and plastic bottles or other containers, as well as in certain environmental clean-up operations. Furthermore, where the composition is to be used as a general industrial oxidation reaction, it may be undesirable to include a surfactant.

The compositions may, in solid form, be storage stable, since it is in general easy to keep the bleach activator and peroxygen donor compound in separate particles and prevent them coming into contact with one another during storage. It is furthermore easier to isolate other components of the composition from one another and from the bleach components, especially where storage sensitive compounds such as enzymes, other biocides or perfumes are present.

Solid compositions may be in the form of particulate mixtures or may be tabletted. Tabletted formulations, or even granular formulations, may include agents to increase the dissolution rate of the compositions upon addition to water. For instance suitable components incorporating into tablets aid disintegration of the tablet. Such ingredients may create effervescence, for instance; a suitable component is sodium bicarbonate, or other alkali metal bicarbonate.

The compositions may also contain ingredients to assist in their application or stability or which improve their appearance, for instance thickeners, dispersants, opacifiers, hydrotropes, dyes, perfumes etc.

The following examples illustrate the invention. In the examples the concentration of peroxygen source is reported in terms of the starting concentration of aqueous hydrogen peroxide to which the other reactants are added. The molar concentration in the mixture can be calculated.

### Example 1 (Reference Example)

### NOBS, BOBS and ISONOBS as activators for peroxygen bleaches at acidic pH, for stains in solution and on fabrics

### 1.1 Experimental

### 1.1.1 Swatches

The activator/peroxygen source combination used was 60% hydrogen peroxide in a 10:1 ratio with the activator. Small swatches of cloth (20-25cm²) were used and the stain was chlorophyll. The bleaching experiments were run using 10mls hydrogen peroxide (60%) which was adjusted to the required pH using sodium hydroxide solution. A weighed quantity of the activator (16.7 mmol) was then added and the mixture stirred for 2 minutes to dissolve the activator. The swatch of cloth was then added and left for 30 minutes with occasional stirring. After 30 minutes, the swatches were removed from the activator solutions, rinsed with deionised water to remove any remaining traces of bleach, dried by application of an electric iron set on the "wool" temperature and the brightness measured using a Hunterlab D25M colorimeter. The pH of the solution was measured after the cloths had been removed. The results are shown in Table 1.

### 1.1.2 The dependence of pH on time

Experiments to monitor the relationship between pH and time were carried out using BOBS and SNOBS, as activators. The pH of 60% hydrogen peroxide was adjusted to about pH 6. To 20mls of this solution was added 33m mols of activator. The pH was measured with time. The results are shown in Table 3.

### 1.1.3 Timed bleaching

Timed bleaching experiments were carried out using the same technique and quantities as in 1.1.2 above with different dwell times of the swatch in the bleach solution. Six separate solutions were prepared and a swatch added to each at the same time. The swatches were removed and rinsed in deionised water after set time periods. The times used were 5mins, 10mins, 20mins, 30mins, lhr and 2hrs. The final brightness after drying by the usual technique was determined using the Hunterlab. The results are shown in Table 2.

### 1.1.4 Time/pH bleaching profile

The solutions and swatches used were prepared as in the above experiments. Four solutions were prepared and a swatch added to each after a set period of time. The cloth was left in the bleach solution for 5mins and then removed and rinsed thoroughly with deionised water. The times at which the swatches were added were after 1min, 15mins, 30mins and 1hr. A different solution was used for each swatch. The activator used was SNOBS. The final brightness after drying of the cloth was measured using the Hunterlab. The results are shown in Table 4.

### 1.1.5 Activation of sodium perborate solutions in the presence of acidifying components.

Two lots of sodium perborate tetrahydrate (20g) mixed with sodium dihydrogen phosphate (17.5g) (to reduce pH) were prepared. To one of these NOBS (4.4g) was added. The mixtures were added to 100mls deionised water and a chlorophyll stained swatch added to each solution. After 1.5hrs the swatch was removed and the brightness measured on the Hunterlab. The pH was measured at timed intervals. The results are given in 1.2.7 below.
1.2.1 The chlorophyll stain was seen to be resilient to bleaching under these harsh conditions which makes it a very good stain to use. The less stain that is removed the better the comparisons which can be drawn between bleaches.
1.2.2 BOBS and NOBS gave the best results. ISONOBS was the least effective. The blank experiments with peracetic acid, water and hydrogen peroxide (at both pH 6 and pH 1) show that activation is occurring, when the activator is present, and that this is not an effect of the lower pH in the activated solutions (Table 1). The drop in pH is good evidence that an acidic species is being produced which is not present in the unactivated peroxide solution.
1.2.3 The decrease in pH on addition of activator was seen to be rapid (Table 3). As would be expected the rate varied with different activators due to differences in both the acid being produced and the rate of perhydrolysis. BOBS was difficult to use, as addition to peroxide produced a thick frothy paste. It was because of this that NOBS was used in subsequent experiments. The problem is probably due to the surfactant properties of these activators, a property which is important in some applications. This increases the wetting ability of the peracid solution.
1.2.4 The bleaching of swatches with different bleaching times showed the expected increase of bleaching with time (Table 2).
1.2.5 The effect of time and pH on the bleaching efficacy of activated solutions was also studied. In this case the dwell time in the bleaching solution was the same (5 mins) but the swatches were added after different times. In four separate solutions cloth was added after lmin, 15mins, 30mins and lhr. Each of these swatches was a quarter of the same larger swatch, to ensure a constant substrate concentration. After 5 minutes in the bleaching solution the cloth was removed and rinsed with deionised water. Comparing of the different swatches, for the same activator, gave a measure of the stability, rate of peracid release and pH dependence of the bleaching. The results are in Table 4. The relationship between these variables is complex but qualitative comparisons can be made.
1.2.6 NOBS was seen to give much better bleaching at all times. The release of peracid seemed to be slow. The cloth added after 1min showed less bleaching than those added later (Table 2). In all cases the stability of the bleach activation with time was remarkably good. There was noticeably better bleaching than with peroxide alone in all cases.
1.2.7 The activation of sodium perborate solutions was also seen to occur under acidic conditions. The use of sodium dihydrogen phosphate enables acidic solutions of perborate to be prepared which also give rise to some degree of buffering (citric acid could alternatively be used). When NOBS was incorporated into such a solution there was noticeably better bleaching than occurred without any added activator. The pH of the solutions was seen to be acidic (pH 6.4 with phosphate) and much more stable than seen with more concentrated peroxide solutions. The pH of the activated and unactivated solutions was very similar.

**Table 1.**

| Activating acidic peroxide with different activators. | | | | | |
|---|---|---|---|---|---|
| Example | Activator | Initial pH | Final pH | Initial Brightness | Final Brightness |
| 2.1 | ISONOBS | 6.00 | 3.22 | 14.26 | 48.5 |
| 2.2 | BOBS | 6.00 | 2.35 | 14.26 | 75.2 |
| 2.3 | SNOBS | 6.00 | 2.58 | 14.26 | 72.4 |
| 2.2 Comp | Blank (H₂O₂₎ | 6.00 | 5.96 | 14.26 | 30.9 |
| 2.3 Comp | Blank (H₂O) | 7.50 | 5.68 | 14.26 | 19.7 |
| 2.4 Comp | Blank (PAAH) | - | - | 14.26 | 59.2 |

**Table 2.**

| The effect of different bleaching times on brightness and solution pH using NOBS. | | |
|---|---|---|
| Time/mins | Final Brightness | pH after 2 hrs |
| 5 | 47.8 | 3.64 |
| 10 | 58.7 | 3.64 |
| 20 | 80.8 | 3.53 |
| 30 | 81.5 | 3.51 |
| 60 | 82.6 | 3.61 |
| 120 | 81.9 | 3.50 |

**Table 3.**

| The effect of activators on solution pH with time. | | |
|---|---|---|
| Time/mins | pH | |
| | BOBS | SNOBS |
| 0 | 6.18 | 6.05 |
| 1 | - | 5.16 |
| 2 | - | 5.01 |
| 3 | 4.77 | - |
| 5 | 4.10 | 4.81 |
| 7 | 3.95 | - |
| 9 | - | - |
| 10 | 3.79 | - |
| 20 | - | - |
| 24 | - | 4.44 |
| 30 | - | - |
| 31 | 3.62 | - |
| 40 | - | 4.18 |
| 65 | - | - |
| 107 | - | 3.95 |
| 1200 | - | - |

**Table 4.**

| Bleaching efficiency against time with SNOBS. | | |
|---|---|---|
| Time/mins | Initial Brightness | Final Brightness |
| 1 | 14.26 | 42.6 |
| 15 | 14.26 | 40.6 |
| 30 | 14.26 | 54.3 |
| 60 | 14.26 | 53.3 |

ISONOBS - Sodium isononanoyloxybenzene sulphonate
NOBS - sodium nonanoyloxybenzene sulphonate
BOBS - benzoyloxybenzoic acid sodium salt

### Example 2 (reference example)

### Biocidal activity of activator/hydrogen peroxide mixtures

2.1 The assessments were performed in a test tube situation following the principles of BS 6471:1984.
   2.1.2 100ml volumes of Nutrient Broth were inoculated with Escherichia coli, Staphylococcus aureus and Streptococcus faecalis.
   2.1.3 A 150mg/l solution of peracetic acid (PAA) was used for comparison. This was prepared in sterile distilled water.
   2.1.4 In order to achieve concentrations comparable with the comparative 150mg/l PAA solution, test solutions of the formulations were prepared using NOBS in the 100ml 1% hydrogen peroxide solution. The amount of activator in the peroxide solution is indicated in the following table.
   2.1.5 1ml of the test bacterial culture was added to 9ml of the appropriate formulation, mixed and left for 5 minutes at room temperature.
   2.1.6 1ml of this liquor was transferred to 9ml of inactivator comprising 50g/l sodium thiosulphate and 0.25g/l catalase in distilled water. The inactivator was filter sterilised using 0.45µm membrane filters.
   2.1.7 From these inactivated liquors, 10-fold serial dilutions were performed using Maximum Recovery Diluent (MRD). Pour plates were prepared using lml volumes of each dilution mixed with molten Plate Count Agar (PCA).
   2.1.8 For controls the procedure was repeated using 1% hydrogen peroxide as the control for the two formulations and sterile distilled water as the control for PAA.
   2.1.9 All plates were incubated for 48 hours at 37°C after which time the number of colonies visible on each plate was counted. The reduction in the number of CFU compared to the control solution is calculated. The results quote the log of the control count/test count. Where the figure is indicated in the table as "more than" a quoted figure, this indicates that the CFU count in the test plate was below the minimum which can be quantified using this technique.
2.2 Results 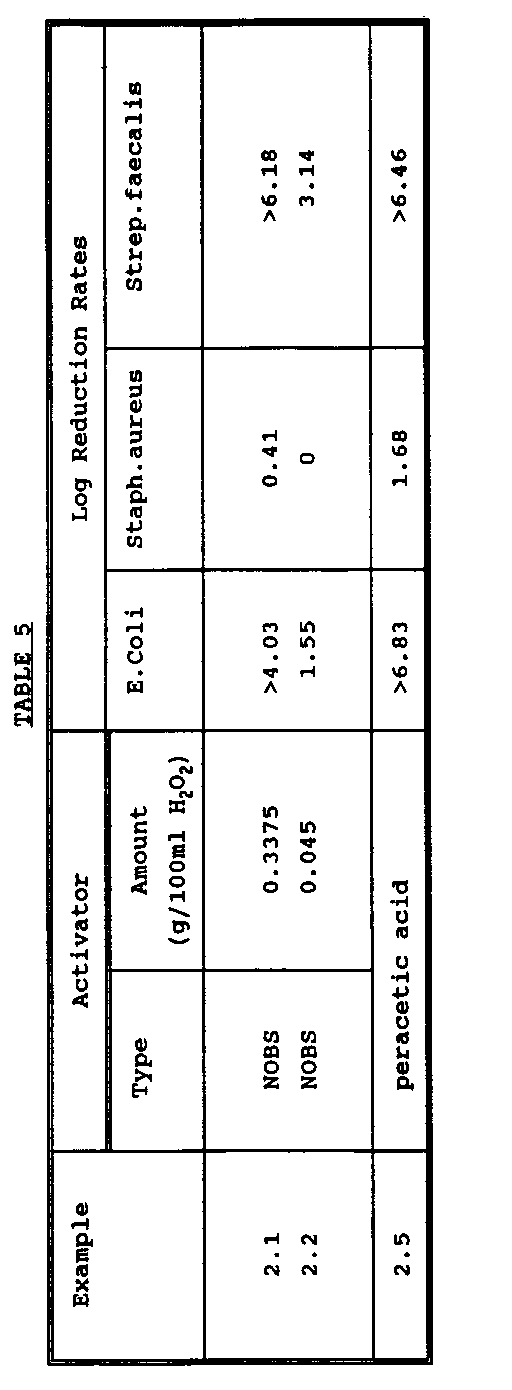
   2.2.1 NOBS/H₂O₂ was found to be effective against all three test organisms. No growth was obtained from the treated cultures of E.coli or Strep. faecalis whilst the number of Staph. aureus colonies recovered was reduced by over half, although a remaining count of 1.5x10³ was still obtained for the higher concentration of NOBS tested. Increasing the concentration of NOBS does seem to have a significant effect of increasing the effectiveness of the peroxide solution.
   2.2.2 PAA was more quicker acting than NOBS/H₂O₂ as, besides a total kill of E.coli and Strep. faecalis, it also reduced the numbers of recovered Staph. aureus by 98%. However, this figure of 98% still left over 3.0x10⁴ cfu/ml recoverable.

### Example 3 (Reference Example)

### The use of penta-acetylglucose (PAG) as activator

3.1 This experiment is to determine the efficacy of PAG, an ester, as a bleach activator under acidic conditions.
3.2 The bleaching experiments were carried out using chlorophyll stained swatches. The three formulations are shown in Table 6. The samples were allowed 75 mins bleaching time. In a further stage to investigate further release of stronger bleach 3 drops 50% w/w NaOH solution were then added to formulations 3.1 and 3.1 comp. A second swatch of material was added and bleached for 25 mins. Formulation 3.3 was at pH 5.6 immediately after NaOH addition (prior to this no bleaching occurred), 3.2 comp showed a pH of 7.5. After 2 hrs pH of 3.3 = 3.11, of 3.2 comp = 6.84.
3.3 The lightness, strength and colour after drying by using an iron on the wool setting were determined by the use of a ICS Texicon Spectra flash 500 (a colorimeter which uses the CIE Lab scale, using version 4.70 software.
3.4 Results

**TABLE 6**

| Example | Solution | Activator | NaOH at 75 min | Lightness | Strength | Colour |
|---|---|---|---|---|---|---|
| 3.1 | 20ml H₂O₂ 60% | 6.51g PAG | - | 0.1 | -2.1 | 1.9 Y |
| 3.1 Comp | 10 ml H₂O₂ 60% | - | - | 1.4 | -0.8 | 0.7 Y |
| 3.2 | 10ml H₂O₂ 10% | 0.65g PAG | - | -0.3 | -1.8 | 1.7 Y |
| 3.3 | 3.1 | | + | 11.1 | -3.9 | 6.5 Y |
| 3.2 Comp | 3.1 Comp | | + | 8.1 | -1.9 | 2.5 Y |

3.5 Y is yellower Positive results in the table indicate lighter, stronger more yellow, respectively.
3.6 The results show that PAG is an effective activator under acidic conditions. The efficiency depends markedly on the pH of the solution. The more strongly acidic the peroxide solution the less effective the activation.

### Example 4

### Strong oxidising agent for Various Activators at pH 6.3

Mixtures comprising 2.58 g sodium perborate monohydrate, 1.58 g sodium bicarbonate and 21 g sodium dihydrogen orthophosphate and 1.88 g of activator were dissolved into 2 litres of water. The concentration of peracid in the solution generated was measured after various periods of time using an iodometric titration. The titration was carried out on ice so as to minimise the reaction rate of hydrogen peroxide in the assay, and determine primarily the stronger bleach released by activator. The results are given in the following table.

**TABLE 7**

| | Relative amount of strong oxidising agent for different activators | |
|---|---|---|
| Time | PAG | SNOBS |
| 5 min | 0.9 | 0.85 |
| 15 min | 0.9 | 1.1 |
| 30 min | 0.6 | 1.3 |
| 45 min | 0.7 | 1.4 |
| 1 hr | 0.4 | 1.4 |
| 1 day | 0.7 | 1.1 |

### Example 5

### Oxidising Agent Concentration for Various Activators at pH 6.3

Mixtures comprising 2.58 g sodium perborate monohydrate, 1.58 g sodium bicarbonate and 21 g sodium dihydrogen orthophosphate and 1.88 g of activator were dissolved into 2 litres of water. The concentration of strong oxidising agent in the solution generated was measured after various periods of time using the iodometric titration mentioned above. The results are given in the following table.

**TABLE 8**

| | Amount of oxidising agent for different activators | |
|---|---|---|
| Time | PAG | SNOBS |
| 5 min | 0.9 | 0.85 |
| 15 min | 0.9 | 1.1 |
| 30 min | 0.6 | 1.3 |
| 45 min | 0.7 | 1.4 |
| 1 hr | 0.4 | 1.4 |
| 1 day | 0.3 | 1.1 |

These results show that the initial release rate for strong oxidising agent by SNOBS is higher than by PAG, both ester activators give good long term release of strong oxidising agent.

## Claims

1. A process in which a solid composite product comprising a solid peroxygen source and an activator compound which is an ester of a C₂ or higher carboxylic acid is added to water whereby the peroxygen source and activator compound are brought into contact with one another in aqueous reaction mixture in which the peroxygen source is present at a concentration of less than 10M whereby the peroxygen source is reacted with the activator compound in a first step in aqueous solution, in the reaction mixture under acidic conditions to form an oxidising species which is a stronger oxidising agent than the peroxygen source and, in a second step, the aqueous composition containing the said oxidising species which is the product of the first step is immediately used without removal of any by-products or addition of any other materials as a bleaching agent, disinfectant, sterilising agent or biocide in an aqueous environment at pH of less than 7.

2. A process according to claim 1 in which the activator comnound has formula I 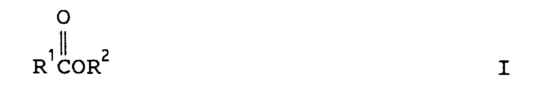 in which R¹ is a alkyl, alkenyl, aralkyl, alkaryl or aryl group, any of which groups has up to 24 carbon atoms and may be substituted or unsubstituted, and R² is selected from C₁₋₂₄-alkyl, -alkenyl, -aralkyl, -alkaryl and -aryl groups, any of which are substituted or unsubstituted, R¹ and R² optionally being joined to form a cyclic group, the peroxygen source being present in the reaction mixture at a concentration of less than 10M in a first step in aqueous solution under acidic conditions to form an oxidising species which is a stonger oxidising agent than the peroxygen source.

3. A process according to claim 2 in which R¹ is selected from methyl and C₄₋₂₄-alkyl and -alkenyl.

4. A process according any preceding claim in which the first step is carried out at a pH less than 6.5, preferably in the range 2.0 to 6.5, more preferably in the range 4.0 to 6.5.

5. A process according to any preceding claim in which water is present in the first step in a molar amount of at least that of the peroxygen source.

6. A process according to any preceding claim in which the composite product is added to the water in an amount such that the concentration of peroxygen source is in the range 0.01 to 10M, preferably in the range 0.1 to 5M, more preferably in the range 0.2 to 2M.

7. Process according to any preceding claim in which the activator is present in an equivalent amount in the range 0.1 to 5 times the peroxide equivalents in the perhydrolysis reaction mixture.

8. A process according to any preceding claim in which the activator is present in the perhydrolysis reaction mixture in an amount less than the stoichiometric amount for reaction with the peroxygen source, preferably in an equivalent amount of at least 0.2 times the peroxide equivalents.

9. A process according to any preceding claim in which the first step is carried out in the presence of an acid-generating species.

10. Process according to claim 9 in which the acid generating species is selected from a polybasic organic carboxylic acid or a compound which drops the pH on reaction with a by-product of the reaction, preferably selected from cis-1,2-diols, eg glycols or polyols, boric acid and sodium dihydrogen phosphate.

11. A process according to any preceding claim in which the temperature of the first step is less than 60°C, preferably less than 50°C.

12. A process according to any preceding claim in which the peroxygen source is selected from urea peroxide, organic peroxides and inorganic persalts.

13. Process according to any preceding claim in which the composite product comprises a single type of particle comprising all of the components of the product.

14. Process according to any preceding claim in which the composite product comprises a surfactant.

15. A composite which is in solid particulate form in the form of blended solid particulates and contains a solid peroxygen source, an activator compound which is an ester of a C₂ or higher carboxylic acid and an acid generating species present in an amount to render acidic a solution in water of the product.

## Patentansprüche

1. Verfahren, in welchem ein festes Kompositprodukt, welches eine feste Persauerstoff-Quelle und eine Aktivator-Verbindung, bei der es sich um einen Ester einer C₂- oder höheren Carbonsäure handelt, umfaßt, zu Wasser gegeben wird, wodurch die Persauerstoff-Quelle und die Aktivator-Verbindung in wäßriger Reaktionsmischung, in welcher die Persauerstoff-Quelle in einer Konzentration von weniger als 10M anwesend ist, miteinander in Kontakt gebracht werden, wodurch die Persauerstoff-Quelle mit der Aktivator-Verbindung in einer ersten Stufe in wäßriger Lösung in der Reaktionsmischung unter sauren Bedingungen umgesetzt wird, um eine oxidierende Spezies zu bilden, bei der es sich um ein stärkeres Oxidationsmittel als die Persauerstoff-Quelle handelt, und in einer zweiten Stufe die diese oxidierende Spezies enthaltende wäßrige Zusammensetzung, die das Produkt der ersten Stufe ist, ohne Entfernung jedweder Nebenprodukte oder Zugabe jedweder anderer Materialien unmittelbar als Bleichmittel, Desinfektionsmittel, Sterilisationsmittel oder Biozid in einer wäßrigen Umgebung bei einem pH von weniger als 7 eingesetzt wird.

2. Verfahren nach Anspruch 1, in welchem die Aktivator-Verbindung die Formel I aufweist 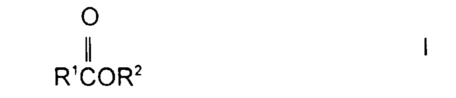 in welcher R¹ eine Alkyl-, Alkenyl-, Aralkyl-, Alkaryl- oder Arylgruppe, die jeweils bis zu 24 Kohlenstoffatome aufweist und substituiert oder unsubstituiert sein kann, ist und R² ausgewählt ist aus C₁₋₂₄-Alkyl-, -Alkenyl-, -Aralkyl-, -Alkaryl- und -Arylgruppen, die jeweils substituiert oder unsubstituiert sind, wobei R¹ und R² gegebenenfalls unter Bildung einer cyclischen Gruppe verbunden sind, wobei die Persauerstoff-Quelle in der Reaktionsmischung bei einer Konzentration von weniger als 10M in einer ersten Stufe in wäßriger Lösung unter sauren Bedingungen anwesend ist, um eine oxidierende Spezies zu bilden, die ein stärkeres Oxidationsmittel als die Persauerstoff-Quelle ist.

3. Verfahren nach Anspruch 2, in welchem R¹ aus Methyl und C₄₋₂₄-Alkyl und -Alkenyl ausgewählt ist.

4. Verfahren nach irgendeinem vorangehenden Anspruch, in welchem die erste Stufe bei einem pH von weniger als 6,5, vorzugsweise im Bereich von 2,0 bis 6,5, bevorzugter im Bereich von 4,0 bis 6,5, durchgeführt wird.

5. Verfahren nach irgendeinem vorangehenden Anspruch, in welchem Wasser in der ersten Stufe in einer Molmenge von mindestens derjenigen der Persauerstoff-Quelle anwesend ist.

6. Verfahren nach irgendeinem vorangehenden Anspruch, in welchem das Kompositprodukt dem Wasser in einer solchen Menge zugegeben wird, daß die Konzentration der Persauerstoff-Quelle im Bereich von 0,01 bis 10M, vorzugsweise im Bereich von 0,1 bis 5M, bevorzugter im Bereich von 0,2 bis 2M, liegt.

7. Verfahren nach irgendeinem vorangehenden Anspruch, in welchem der Aktivator in einer Äquivalentmenge im Bereich vom 0,1- bis 5-fachen der Peroxid-Äquivalente in der Perhydrolyse-Reaktionsmischung anwesend ist.

8. Verfahren nach irgendeinem vorangehenden Anspruch, in welchem der Aktivator in der Perhydrolyse-Reaktionsmischung in einer Menge, die geringer als die stöchiometrische Menge für die Umsetzung mit der Persauerstoff-Quelle ist, vorzugsweise in einer Äquivalentmenge vom mindestens 0,2-fachen der Peroxid-Äquivalente, anwesend ist.

9. Verfahren nach irgendeinem vorangehenden Anspruch. in welchem die erste Stufe in Anwesenheit einer säureerzeugenden Spezies durchgeführt.

10. Verfahren nach Anspruch 9, in welchem die säureerzeugende Spezies aus einer mehrbasigen organischen Carbonsäure oder einer Verbindung, die den pH bei Reaktion mit einem Nebenprodukt der Reaktion absenkt, vorzugsweise ausgewählt aus cis-1,2-Diolen, z.B. Glycolen oder Polyolen, Borsäure und Natriumdihydrogenphosphat, ausgewählt wird.

11. Verfahren nach irgendeinem vorangehenden Anspruch, in welchem die Temperatur der ersten Stufe weniger als 60°C, vorzugsweise weniger als 50°C, beträgt.

12. Verfahren nach irgendeinem vorangehenden Anspruch, in welchem die Persauerstoff-Quelle aus Harnstoffperoxid, organischen Peroxiden und anorganischen Persalzen ausgewählt wird.

13. Verfahren nach irgendeinem vorangehenden Anspruch, in welchem das Kompositprodukt einen einzigen Typ von Teilchen, der alle Komponenten des Produkts umfaßt, umfaßt.

14. Verfahren nach irgendeinem vorangehenden Anspruch, in welchem das Kompositprodukt ein Tensid umfaßt.

15. Komposit, das in fester teilchenförmiger Form in Form von gemischten festen teilchenförmigen Stoffen vorliegt und eine feste Persauerstoff-Quelle, eine Aktivator-Verbindung, bei der es sich um einen Ester einer C₂- oder höheren Carbonsäure handelt, und eine säureerzeugende Spezies, die in einer Menge anwesend ist, um eine Lösung des Produkts in Wasser sauer zu machen, enthält.

## Revendications

1. Procédé dans lequel un produit composite solide comprenant une source de peroxygène solide et un composé activateur qui est un ester d'un acide carboxylique en C₂ ou plus est ajouté à de l'eau, grâce à quoi la source de peroxygène et le composé activateur sont mis en contact l'un avec l'autre dans un mélange réactionnel aqueux dans lequel la source de peroxygène est présente en une concentration inférieure à 10 M, grâce à quoi la source de peroxygène réagit avec le composé activateur dans une première étape en solution aqueuse, dans le mélange réactionnel dans des conditions acides pour former un composé oxydant qui est un agent oxydant plus fort que la source de peroxygène et, dans une deuxième étape, la composition aqueuse contenant ledit composé oxydant qui est le produit de la première étape est immédiatement utilisée sans élimination de tous sous-produits ou addition de tous autres matériaux en tant qu'agent de blanchiment, désinfectant, agent stérilisant ou biocide dans un environnement aqueux à un pH inférieur à 7.

2. Procédé selon la revendication 1, dans lequel le composé activateur répond à la formule I : 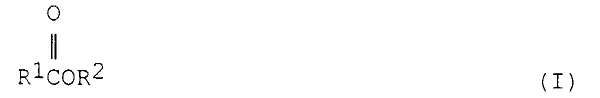 dans laquelle R¹ est un groupe alkyle, alcényle, aralkyle, alcaryle ou aryle, ces groupes ayant jusqu'à 24 atomes de carbone et pouvant être éventuellement substitués, et R² est choisi parmi les groupes alkyle, alcényle, aralkyle, alcaryle et aryle en C₁ à C₂₄, éventuellement substitués, R¹ et R² étant éventuellement réunis pour former un groupe cyclique, la source de peroxygène étant présente dans le mélange réactionnel à une concentration inférieure à 10 M dans une première étape en solution aqueuse dans des conditions acides pour former un composé oxydant qui est un agent oxydant plus fort que la source de peroxygène.

3. Procédé selon la revendication 2, dans lequel R¹ est choisi parmi les groupes méthyle et alkyle et alcényle en C₄ à C₂₄.

4. Procédé selon l'une quelconque des revendications précédentes, dans lequel la première étape se déroule à un pH inférieur à 6,5, de préférence compris entre 2,0 et 6,5, mieux encore compris entre 4,0 et 6,5.

5. Procédé selon l'une quelconque des revendications précédentes, dans lequel de l'eau est présente dans la première étape en une quantité molaire au moins égale à celle de la source de peroxygène.

6. Procédé selon l'une quelconque des revendications précédentes, dans lequel le produit composite est ajouté à l'eau en une quantité telle que la concentration de source de peroxygène soit comprise entre 0,01 et 10 M, de préférence entre 0,1 et 5 M, mieux encore entre 0,2 et 2 M.

7. Procédé selon l'une quelconque des revendications précédentes, dans lequel l'activateur est présent en une quantité équivalente représentant de 0,1 à 5 fois les équivalents peroxyde dans le mélange réactionnel de perhydrolyse.

8. Procédé selon l'une quelconque des revendications précédentes, dans lequel l'activateur est présent dans le mélange réactionnel de perhydrolyse en une quantité inférieure à la quantité stoechiométrique pour la réaction avec la source de peroxygène, de préférence en une quantité équivalente représentant au moins 0,2 fois les équivalents peroxyde.

9. Procédé selon l'une quelconque des revendications précédentes, dans lequel la première étape se déroule en présence d'un composé générateur d'acide.

10. Procédé selon la revendication 9, dans lequel le composé générateur d'acide est choisi parmi les acides carboxyliques organiques polybasiques ou les composés qui font chuter le pH lors de la réaction avec un sous-produit de la réaction, et est de préférence choisi parmi les cis-1,2-diols, par exemple les glycols ou les polyols, l'acide borique et le dihydrogénophosphate de sodium.

11. Procédé selon l'une quelconque des revendications précédentes, dans lequel la température de la première étape est inférieure à 60°C, de préférence inférieure à 50°C.

12. Procédé selon l'une quelconque des revendications précédentes, dans lequel la source de peroxygène est choisie parmi le peroxyde d'urée, les peroxydes organiques et les persels minéraux.

13. Procédé selon l'une quelconque des revendications précédentes, dans lequel le produit composite comprend un type unique de particules comprenant tous les composants du produit.

14. Procédé selon l'une quelconque des revendications précédentes, dans lequel le produit composite comprend un tensioactif.

15. Composite, qui a une forme particulaire solide, sous la forme de particules solides mélangées, et qui contient une source de peroxygène solide, un composé activateur qui est un ester d'un acide carboxylique en C₂ ou plus et un composé générateur d'acide, présent en une quantité telle qu'elle rende acide une solution dans l'eau du produit.
